# EUROPEAN PATENT APPLICATION

(11) **EP 4 480 474 A1**
(43) Date of publication of application: **25.12.2024**
(21) Application number: 23796357.4
(22) Date of filing: 25.04.2023
(51) Int. Cl.: A61K 31/18, A61K 31/198, A61K 45/00, A61P 25/16

(54) **PHARMACEUTICAL COMPOSITION FOR TREATMENT OF PARKINSON'S DISEASE**

(30) Priority: 27.04.2022 JP 2022073291
(71) Applicant: Eisai R&D Management Co., Ltd., Tokyo 112-8088 (JP)
(72) Inventor: FUKUSHIMA Kazuyuki, Tsukuba-shi, Ibaraki 300-2635 (JP)
(74) Representative: Hoffmann Eitle
(86) International application number: PCT/JP2023/016215
(87) International publication number: WO 2023/210617

(57) **Abstract**

Disclosed is a pharmaceutical composition for treatment of Parkinson's disease comprising N-[(1S)-2,2,5,7-tetrafluolo-2,3-dihydro-1H-inden-1-yl]sulfamide represented by formula (1) or a pharmaceutically acceptable salt thereof.

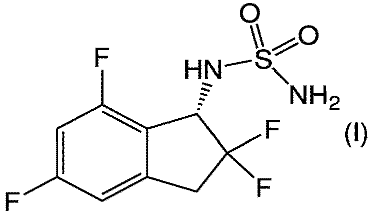

## Description

### Technical Field

The present disclosure relates to a pharmaceutical composition for treating Parkinson's disease.

### Background Art

Parkinson's disease is a neurodegenerative disease caused by selective lesion of dopaminergic neurons in the substantia nigra. Patients with Parkinson's disease experience progressive motor symptoms and non-motor symptoms, and both symptoms greatly deteriorate the qualities of lives (QOLs) of the patients. As the motor symptoms, bradykinesia, tremor, walking difficulty, dysphonia, muscle rigidity, muscle weakness, loss of facial expression, postural deformity, and the like are developed in the early stage of the onset; and dyskinesia, motor fluctuation, frozen gait, fall, and the like are developed in the advanced stage of Parkinson's disease. As the non-motor symptoms, olfactory dysfunction, sleep disorder, constipation, erectile dysfunction, and the like are developed (Non Patent Literature 1).

The basis of the treatment of Parkinson's disease is pharmacotherapy centered on dopamine replacement therapy, including the administration of levodopa (L-dopa), which is a dopamine precursor. Although levodopa is used as a main medicament for dopamine replacement therapy, it is known that the therapeutic effect thereof on some motor symptoms is insufficient, and it is known that motor complications such as dyskinesia and a wearing-off phenomenon develop with a long-term medication or the progress of the disease. Levodopa is not only administered alone, but also used in combination with a dopa decarboxylase inhibitor such as carbidopa or benserazide; a dopamine receptor agonist; a monoamine oxidase (MAOB) inhibitor; a catechol-O-methyltransferase (COMT) inhibitor such as entacapone; or the like (Non Patent Literatures 1 and 2).

γ-Aminobutyric acid (GABA) is a main inhibitory neurotransmitter in the central nervous system. GABA released into synaptic clefts acts on GABA_{A} receptors or GABA_{B} receptors, and is then taken up by presynaptic cells and the like through GABA transporters (GATs). It has been reported that GAT-1, which is a subtype of GATs, is associated with Parkinson's disease. It is disclosed in Non Patent Literature 3 that, in a mouse model of early parkinsonism, GAT-1 is downregulated to enhance the tonic GABAergic inhibition of dopamine release, and nigrostriatal co-release of GABA is attenuated. It is disclosed in Non Patent Literature 4 that while the blockage of GAT-1 in lateral habenular nuclei improves depression-like behavior in a Parkinson's disease rat model, the blockage does not influence spontaneous movement. It is disclosed in Non Patent Literature 5 that pre-onset treatment of Parkinson's disease with tiagabine, which is GAT-1 inhibitor, attenuates microglial activation, and in a Parkinson's disease mouse model, partially protects nigrostriatal axis to ameliorate motor dysfunction.

N-[(1S)-2,2,5,7-tetrafluoro-2,3-dihydro-1H-inden-1-yl]sulfamide (hereinafter also referred to as "Compound (I)"), represented by formula (I), has been reported to have a higher therapeutic index (TD₅₀/ED₅₀), which is calculated by comparing the anti-seizure effect (ED₅₀) in a mouse kindling model, which is an epilepsy model, with the neurotoxic effect (TD₅₀) in the rotarod test as compared with a control compound and to have availability as a safer therapeutic agent for epilepsy (Patent Literature 1). Compound (I) has been reported to exhibit an analgesic effect in various animal models, and have availability as a therapeutic and/or preventive agent for pain (Patent Literature 2). Furthermore, it is also reported that the combined use of Compound (I) and an AMPA glutamate receptor antagonist is expected to provide a safe and effective pharmacotherapy for all the cases of epilepsy (Patent Literature 3). The relationship between Compound (I) and the treatment of Parkinson's disease has not, however, been reported yet.

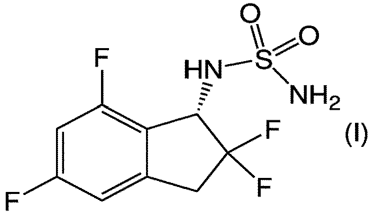

### Citation List

### Patent Literature

Patent Literature 1: U.S. Patent No. 9018260
Patent Literature 2: U.S. Patent No. 9610262
Patent Literature 3: U.S. Patent Application Publication No. 2021/000771
Patent Literature 4: WO 2003/063876

### Non Patent Literature

Non Patent Literature 1: Lee et al, "A review on Parkinson's disease treatment", Neuroimmunol. Neuroinflammation, 2021, 8:222-44.
Non Patent Literature 2: Bastiaan R. Bloem et al, "Parkinson's disease", Lancet, 2021, 397: 2284-303.
Non Patent Literature 3: Roberts et al, "GABA uptake transporters support dopamine release in dorsal striatum with maladaptive downregulation in a parkinsonism model", Nature Communications, 2020, 11:4958.
Non Patent Literature 4: Lyu et al, "Blockade of GABA transporter-1 and GABA transporter-3 in the lateral habenula improves depressive-like behaviors in a rat model of Parkinson's disease", Neuropharmacology, 2020, 181: 108369.
Non Patent Literature 5: Liu et al, "Tiagabine Protects Dopaminergic Neurons against Neurotoxins by Inhibiting Microglial Activation", Scientific Reports, 2015, 5:15720.

### Summary of Invention

### Technical Problem

An object of the present invention is to provide a pharmaceutical composition for treating Parkinson's disease.

### Solution to Problem

The present inventors have diligently studied to achieve the above-mentioned object and found that Compound (I) improves symptoms, especially motor symptoms, of Parkinson's disease in a Parkinson's disease primate model. It has also been found that the combination of Compound (I) and a dopamine-replacement-therapeutic agent is also effective in improving symptoms, especially motor symptoms of Parkinson's disease.

That is, the present disclosure relates to the following <1> to <27>.
<1> A pharmaceutical composition for treating Parkinson's disease, comprising Compound (I): 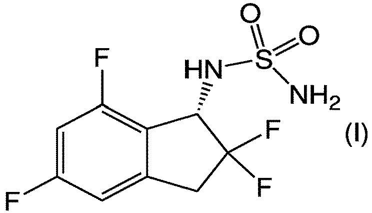 or a pharmaceutically acceptable salt thereof.
<2> The pharmaceutical composition according to <1>, comprising Compound (I) or the pharmaceutically acceptable salt thereof, wherein the composition is administered in combination with a dopamine-replacement-therapeutic agent.
<3> The pharmaceutical composition according to <2>, wherein the dopamine-replacement-therapeutic agent comprises levodopa.
<3-1> The pharmaceutical composition according to <2> or <3>, wherein the dopamine-replacement-therapeutic agent, and the Compound (I) or the pharmaceutically acceptable salt thereof are administered simultaneously, separately, sequentially, or at an interval.
<4> A pharmaceutical composition for treating Parkinson's disease, comprising levodopa, wherein the composition is administered in combination with Compound (I) or a pharmaceutically acceptable salt thereof.
<4-1> The pharmaceutical composition according to <4>, wherein the levodopa, and the Compound (I) or the pharmaceutically acceptable salt thereof are administered simultaneously, separately, sequentially, or at an interval.
<5> The pharmaceutical composition according to any one of <1> to <4-1>, wherein the composition is for treating a motor symptom of Parkinson's disease.
<6> A method of treating Parkinson's disease, comprising administering Compound (I) or a pharmaceutically acceptable salt thereof to a patient in need thereof.
<7> The method according to <6>, further comprising administering a dopamine-replacement-therapeutic agent.
<8> The method according to <7>, wherein the dopamine-replacement-therapeutic agent comprises levodopa.
<8-1> The method according to <7> or <8>, wherein the dopamine-replacement-therapeutic agent, and Compound (I) or the pharmaceutically acceptable salt thereof are administered simultaneously, separately, sequentially, or at an interval.
<9> The method according to any one of <6> to <8-1>, wherein the method is of treating a motor symptom of Parkinson's disease.
<10> A therapeutic agent for Parkinson's disease, comprising Compound (I) or a pharmaceutically acceptable salt thereof.
<11> The therapeutic agent according to <10>, comprising Compound (I) or the pharmaceutically acceptable salt thereof, wherein the agent is administered in combination with a dopamine-replacement-therapeutic agent.
<12> The therapeutic agent according to <11>, wherein the dopamine-replacement-therapeutic agent comprises levodopa.
<12-1> The therapeutic agent according to <11> or <12>, wherein the dopamine-replacement-therapeutic agent, and Compound (I) or the pharmaceutically acceptable salt thereof are administered simultaneously, separately, sequentially, or at an interval.
<13> A therapeutic agent for Parkinson's disease, comprising levodopa, wherein the agent is administered in combination with Compound (I) or the pharmaceutically acceptable salt thereof.
<13-1> The therapeutic agent according to <13>, wherein levodopa, and the Compound (I) or the pharmaceutically acceptable salt thereof are administered simultaneously, separately, sequentially, or at an interval.
<14> The therapeutic agent according to any one of <10> to <13-1> for treating a motor symptom of Parkinson's disease.
<15> Use of Compound (I) or a pharmaceutically acceptable salt thereof for the manufacture of a therapeutic agent for Parkinson's disease.
<16> The use according to <15>, wherein the therapeutic agent for Parkinson's disease is administered in combination with a dopamine-replacement-therapeutic agent.
<17> The use according to <16>, wherein the dopamine-replacement-therapeutic agent comprises levodopa.
<17-1> The use according to <16> or <17>, wherein the dopamine-replacement-therapeutic agent, and the Compound (I) or the pharmaceutically acceptable salt thereof are administered simultaneously, separately, sequentially, or at an interval.
<18> Use of levodopa for the manufacture of a therapeutic agent for Parkinson's disease to be administered in combination with Compound (I) or the pharmaceutically acceptable salt thereof.
<18-1> The use according to <18>, wherein levodopa, and the Compound (I) or the pharmaceutically acceptable salt thereof are administered simultaneously, separately, sequentially, or at an interval.
<19> The use according to any one of <15> to <18-1>, for treating a motor symptom of Parkinson's disease.
<20> Compound (I) or a pharmaceutically acceptable salt thereof, for use in treating Parkinson's disease.
<21> The compound or the salt thereof according to <20>, wherein the compound or the salt thereof is administered in combination with a dopamine-replacement-therapeutic agent.
<22> The compound or the salt thereof according to <21>, wherein the dopamine-replacement-therapeutic agent comprises levodopa.
<23> The Compound (I) or the pharmaceutically acceptable salt thereof according to <21> or <22>, wherein the dopamine-replacement-therapeutic agent, and the Compound (I) or the pharmaceutically acceptable salt thereof are administered simultaneously, separately, sequentially, or at an interval.
<24> The Compound (I) or the pharmaceutically acceptable salt thereof according to any one of <20> or <23>, for treating a motor symptom of Parkinson's disease.
<25> A dopamine-replacement-therapeutic agent for use in treating Parkinson's disease, wherein the agent is administered in combination with Compound (I) or the pharmaceutically acceptable salt thereof.
<25-1> The dopamine-replacement-therapeutic agent according to <25>, comprising levodopa.
<25-2> The dopamine-replacement-therapeutic agent according to <25> or <25-1>, wherein the dopamine-replacement-therapeutic agent, and the Compound (I) or the pharmaceutically acceptable salt thereof are administered simultaneously, separately, sequentially, or at an interval.
<26> The dopamine-replacement-therapeutic agent according to any one of <25> to <25-2>, for treating a motor symptom of Parkinson's disease.
<27> A combination of a dopamine-replacement-therapeutic agent and Compound (I) or a pharmaceutically acceptable salt thereof, for use in treating Parkinson's disease.

### Advantageous Effects of Invention

A pharmaceutical composition for treating Parkinson's disease of the present invention has availability in the treatment of Parkinson's disease such as, for example, improvement in motor symptoms.

### Brief Description of Drawings

Figure 1 is a graph showing the results of the total scores of NDSs (neurological deficit scores) in Test Example 1.
Figure 2 is a graph showing the results of the scores of generalized behaviors in Test Example 1.
Figure 3 is a graph showing the results of the scores of specific motor functions in Test Example 1.
Figure 4 is a graph showing the results of tremor scores in Test Example 1.
Figure 5 is a graph showing the results of the total scores of NDSs in Test Example 2.
Figure 6 is a graph showing the results of the scores of generalized behaviors in Test Example 2.
Figure 7 is a graph showing the results of the scores of specific motor functions in Test Example 2.
Figure 8 is a graph showing the results of tremor scores in Test Example 2.
Figure 9 is a graph showing the results of dyskinesia scores in Test Example 3.

### Description of Embodiments

Hereinafter, the contents of the present disclosure will be described in detail.

N-[(1S)-2,2,5,7-tetrafluoro-2,3-dihydro-1H-inden-1-yl]sulfamide is a compound represented by the following formula (I).

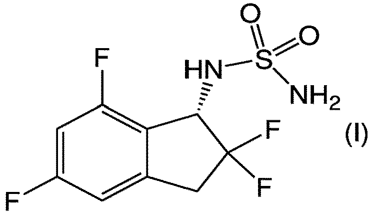

For example, Compound (I) or a pharmaceutically acceptable salt thereof can be prepared by a method described in Patent Literature 1.

The "pharmaceutically acceptable salt" is not particularly limited as long as the salt is formed by Compound (I) and another substance. For example, the pharmaceutically acceptable salt can be an acid addition salt such as an inorganic acid salt, an organic acid salt, an acidic amino acid salt, and the like.

Examples of the salt with an inorganic acid include a salt with hydrochloric acid, hydrobromic acid, sulfuric acid, nitric acid, phosphoric acid, and the like. Examples of the salt with an organic acid include a salt with acetic acid, succinic acid, fumaric acid, maleic acid, tartaric acid, citric acid, lactic acid, stearic acid, benzoic acid, methanesulfonic acid, ethanesulfonic acid, p-toluenesulfonic acid, and the like.

Examples of the salt with an acidic amino acid include a salt with aspartic acid, glutamic acid, and like.

The pharmaceutical composition for treating Parkinson's disease of the present disclosure can be produced by mixing pharmaceutically acceptable additives with Compound (I) or a pharmaceutically acceptable salt thereof. The pharmaceutical composition for treating Parkinson's disease of the present disclosure can be produced, for example, in accordance with a known method, such as the method described in the General Rules for Preparations of the 18th revised Japanese Pharmacopeia.

The pharmaceutical composition for treating Parkinson's disease of the present disclosure can be suitably administered to a patient depending on the dosage form thereof.

The pharmaceutical composition for treating Parkinson's disease of the present disclosure can be orally administered in the form of a solid preparation, such as a tablet, granules, fine granules, powders, or capsules; solution; jelly; syrup; or the like. The pharmaceutical composition may be parenterally administered in the form of an injection, a suppository, ointment, a poultice, or the like.

For producing a solid preparation, the preparation can be produced by (i) adding an excipient; and, if necessary, a binder, a disintegrator, a lubricant, a coloring agent, a flavoring agent and the like, as additives to the salt of Compound (I) or a crystal thereof, and then (ii) forming a tablet, granules, fine granules, dusting powder, a capsule, or the like by an ordinary method. The above-mentioned additives can be appropriately combined for formulation. The tablet, the granules, and the like may be coated as needed.

Examples of the excipient include lactose, sucrose, glucose, cornstarch, mannitol, sorbitol, starch, pregelatinized starch, dextrin, crystalline cellulose, calcium hydrogen phosphate, and the like.

Examples of the binder include methyl cellulose, ethyl cellulose, gum arabic, hydroxypropyl methylcellulose, hydroxypropyl cellulose, and the like.

Examples of the disintegrator include low-substituted hydroxypropyl cellulose, carboxymethyl cellulose, calcium carboxymethyl cellulose, croscarmellose sodium, sodium carboxymethyl starch, crospovidone, and the like.

Examples of the lubricant include talc, silica, magnesium stearate, calcium stearate, sodium stearyl fumarate, polyethylene glycol, and the like.

Examples of the coloring agent include diiron trioxide, yellow diiron trioxide, carmine, β-carotin, titanium oxide, riboflavin sodium phosphate, yellow aluminum lake, cochineal, and the like.

Examples of the flavoring agent include cocoa powder, ascorbic acid, tartaric acid, peppermint oil, borneol, powdered cinnamon bark, and the like.

For producing an injection, the injection can be produced by (i) as needed, adding a pH adjustor, a buffering agent, a suspending agent, a solubilizer, a stabilizer, an isotonizing agent, a preservative, and the like to the main ingredient and (ii) preparing an intravenous, subcutaneous, or intramuscular injection or an intravenous drip by an ordinary method. In this case, the injection can be freeze-dried by an ordinary method as needed.

Examples of the pH adjustor and the buffering agent include hydrochloric acid, sodium carbonate, sodium hydrogen carbonate, citric acid, sodium citrate, sodium dihydrogen citrate, glycine, phosphoric acid, sodium dihydrogen phosphate, sodium monohydrogen phosphate, sodium hydroxide, acetic acid, sodium acetate, meglumine, and the like.

Examples of the suspending agent include sodium alginate, sucrose fatty acid ester, polysorbate 80, gum arabic, powdered tragacanth, polyoxyethylene sorbitan monolaurate, and the like.

Examples of the solubilizer include polyoxyethylene hydrogenated castor oil, polysorbate 80, nicotinamide, polyoxyethylene sorbitan monolaurate, glycerin fatty acid ester, polyethylene glycol, propylene glycol, benzyl benzoate, ethanol, triethanolamine, and the like.

Examples of the stabilizer include sodium sulfite, sodium metabisulfite, and the like.

Examples of the isotonizing agent include glucose, mannitol, sorbitol, and the like.

Examples of the preservative include methyl para-oxybenzoate, ethyl para-oxybenzoate, sorbic acid, phenol, cresol, chlorocresol, and the like.

In the pharmaceutical composition for treating Parkinson's disease of the present disclosure, the dose of Compound (I) or the pharmaceutically acceptable salt thereof varies depending on severity of the symptom; age, gender, body weight, and sensitivity difference of a patient; administration method; administration timing; and type of the pharmaceutical preparation. In a case of oral administration to an adult (with a body weight of 60 kg), usually 100 µg to 5 g, 300 µg to 3 g, as one aspect, or 1 mg to 1 g, as another aspect, of Compound (I) or the pharmaceutically acceptable salt thereof is administered per day in a single dose or several divided doses. In a case of the administration by injection, 30 µg to 1 g, 30 µg to 500 mg, as one aspect, or 50 µg to 300 mg, as another aspect, of Compound (I) or the pharmaceutically acceptable salt thereof is administered per day in a single dose or several divided doses. In one aspect, Compound (I) or the pharmaceutically acceptable salt thereof is orally administered in an amount of 20 mg to 180 mg, 40 mg to 160 mg, 20 mg to 80 mg, 20 mg to 60 mg, 20 mg, 40 mg, 60 mg, 80 mg, 100 mg, 120 mg, 140 mg, 160 mg, or 180 mg once a day.

The pharmaceutical composition for treating Parkinson's disease of the present disclosure may be administered in combination with a dopamine-replacement-therapeutic agent. The dopamine-replacement-therapeutic agent refers to a medicament to be used in dopamine replacement therapy. The dopamine replacement therapy refers to a therapy which increases the level of dopamine or a therapy which stimulates a dopamine receptor directly. In other word, the dopamine-replacement-therapeutic agent can be a medicament which increases the level of dopamine or a medicament which stimulates a dopamine receptor directly. For example, the dopamine-replacement-therapeutic agent can be at least one selected from the group consisting of a dopamine precursor, dopa decarboxylase inhibitor, catechol-O-methyltransferase (COMT) inhibitor, monoamine oxidase (MAOB) inhibitor, a dopamine release-accelerating agent, or a dopamine agonist. For example, the dopamine precursor includes levodopa and the like. Examples of the dopa decarboxylase inhibitor can be carbidopa, benserazide, or the like. For example, the COMT inhibitor can be entacapone or the like. For example, the MAOB inhibitor can be safinamide, selegiline, rasagiline, or the like. For example, the dopamine release-accelerating agent can be amantadine or the like. For example, the dopamine agonist can ne cabergoline, bromocriptine, pergolide, talipexole, pramipexole, ropinirole, rotigotine, or apomorphine. These medicaments may be in the form of a pharmaceutically acceptable salt.

The dopamine-replacement-therapeutic agent in the present disclosure may be one kind or a combination of two or more kinds. Examples of the combination of two or more kinds of dopamine-replacement-therapeutic agents include a combined use of a dopamine precursor and a dopa decarboxylase inhibitor (for example, a combined use of levodopa and carbidopa and the combined use of levodopa and benserazide); a combined use of a dopamine precursor, a dopa decarboxylase inhibitor, and a COMT inhibitor (for example, a combined use of levodopa, carbidopa, and entacapone); a combined use of a dopamine precursor and a dopamine agonist (for example, a combined use of levodopa and talipexole, a combined use of levodopa and cabergoline, and a combined use of levodopa and ropinirole); a combined use of a dopamine precursor and a MAOB inhibitor (for example, a combined of levodopa and selegiline); and the like. In one aspect, the dopamine-replacement-therapeutic agent contains levodopa. The dopamine-replacement-therapeutic agent may be levodopa alone or a combination with other one kind or two or more kinds of drug[s] used in dopamine replacement therapy.

The dopamine-replacement-therapeutic agent and the dose thereof can be determined by referring to clinical guidelines on Parkinson's disease, for example, "Pakinson Byo Shiryo Gaidorain 2018 (Parkinson's disease clinical guidelines 2018)", supervised the Japanese Society of Neurology, (IGAKU-SHOIN Ltd.).

For example, the single-drug dose of levodopa varies depending on the severity of the symptom, the age, the gender, the body weight, and the sensitivity difference of a patient, the administration method, the administration time, and the type of the pharmaceutical preparation. In the case of oral administration to an adult (with a body weight of 60 kg), levodopa is usually administered in an amount of 200 mg to 600 mg per day in a single dose or several divided doses, the amount is gradually increased by 200 mg to 400 mg per day every 2 to 3 days, and 2 to 4 weeks later, levodopa is administered in an amount of 2000 mg to 3600 mg per day as a maintenance dose. The dose of a combination drug of levodopa/carbidopa (at a mass ratio of 100:10) varies depending on the severity of the symptom, the age, the gender, the body weight, and the sensitivity difference of a patient, the administration method, the administration time, and the type of the pharmaceutical preparation. In the case of oral administration to an adult (with a body weight of 60 kg), the combination drug is usually administered in an amount of 100 mg to 300 mg per day as levodopa in a single dose or several divided doses, the amount as levodopa is increased by 100 mg to 125 mg per day every day or every other day, and the combination drug is administered in an amount of 500 mg to 1000 mg per day as levodopa as a maintenance dose 1 to 2 weeks later. The dose of a combination drug of levodopa/carbidopa (at a mass ratio of 100:25) varies depending on the severity of a symptom, the age, the gender, the body weight, and the sensitivity difference of a patient, the administration method, the administration time, and the type of the pharmaceutical preparation. In the case of the oral administration to an adult (with a body weight of 60 kg), the combination drug is usually administered in an amount of 200 mg to 300 mg per day as levodopa in several divided doses, the amount as levodopa is increased by 100 mg to 200 mg per day every day or every other day as needed, and the combination drug is administered in an amount of 400 mg to 1600 mg per day as levodopa as the final maintenance dose. In the case of the nasojejunal or gastrojejunal administration, the combination drug is administered in an amount of 100 mg to 200 mg as levodopa over 10 to 30 minutes in the morning and then continuously administered in an amount of 40 mg to 120 mg hourly as levodopa, and can be additionally administered in an amount of 10 mg to 40 mg per dose as levodopa as necessary so that the maximum duration of administration is 16 hours per day, and the total dose per day does not exceed 2000 mg as levodopa. The dose of a combination drug of levodopa/benserazide (at a mass ratio of 100:25) varies depending on the severity of the symptom, the age, the gender, the body weight, and the sensitivity difference of a patient, the administration method, the administration time, and the type of the pharmaceutical preparation. In the case of the oral administration to an adult (with a body weight of 60 kg), the combination drug is usually administered in an amount of 100 mg to 300 mg per day as levodopa in a single dose or several divided doses, the amount is gradually increased by 100 mg to 200 mg per day every 2 to 3 days, and the combination drug is administered in an amount of 300 mg to 600 mg per day as a maintenance dose 1 to 3 weeks later. The dose of a combination drug of levodopa/carbidopa/entacapone varies depending on the severity of the symptom, the age, the gender, the body weight, and the sensitivity difference of a patient, the administration method, the administration time, and the type of the pharmaceutical preparation. In the case of the oral administration to an adult (with a body weight of 60 kg), the combination drug is usually administered in an amount of 50 mg to 200 mg as levodopa several times per day so that the total daily amount as levodopa does not exceed 1500 mg, the total daily amount as carbidopa does not exceed 300 mg, the total daily amount as entacapone does not exceed 1600 mg, and the number of doses does not exceed eight per day.

The administration form of a drug to be used in the case of the combination of Compound (I) or a pharmaceutically acceptable salt thereof with the dopamine-replacement-therapeutic agent in the present disclosure is not particularly limited, and Compound (I) or a pharmaceutically acceptable salt thereof and dopamine-replacement-therapeutic agent only have to be administered in combination. For example, Compound (I) or a pharmaceutically acceptable salt thereof and the dopamine-replacement-therapeutic agent are administered to a patient simultaneously, separately, sequentially, or at an interval. The term "simultaneously" as used here means that components are administered in the same period or strictly simultaneously, or in the identical route of administration. The term also means that the components are administered without a considerable interval between the both so that each component can exert a desired effect. The term "separately" means that the components are administered at different intervals or with different frequencies, or in different routes of administration. The term "sequentially" means that the components are administered in the identical route of administration or in different routes of administration within a certain period in any order. The term "at an interval" means that the components are administered in the identical route of administration or in different routes of administration at an interval. Compound (I) or a pharmaceutically acceptable salt thereof, and the dopamine-replacement-therapeutic agent may be administered, for example, as the identical preparation or as different preparations.

The patient to be treated in the present disclosure is a patient suffering Parkinson's disease, for example, a patient suffering Parkinson's disease accompanied with motor symptoms and/or a patient suffering Parkinson's disease accompanied with motor complications. Examples of the motor symptoms include tremor, bradykinesia, muscle rigidity (muscular rigidity), postural deformity, and the like.

The pharmaceutical composition in the present disclosure does not exacerbate motor complications that accompanies the progression of Parkinson's disease or does not influence the motor complications. Examples of the motor complications include wearing off, dyskinesia, and dystonia. In one aspect, the motor complications are dyskinesia.

If the pharmaceutical composition in the present disclosure is administered in combination with the dopamine-replacement-therapeutic agent such as levodopa, a reduction in the dose of the dopamine-replacement-therapeutic agent achieves the prevention of the exacerbation of motor complications accompanying the progress of the Parkinson's disease or the influence on motor complications, and a therapeutic effect equal to or greater than the effect without reduction of the dose of the dopamine-replacement-therapeutic agent is meanwhile exhibited on the motor symptoms.

### Examples

### Pharmacological Test Example

The therapeutic effect of Compound (I) on Parkinson's disease was examined using Parkinson's disease model animals which are produced by administering MPTP (1-methyl-4-phenyl-1,2,3,6-tetrahydropyridine).

### Test Example 1: Therapeutic effect of administration of Compound (I) alone

MPTP was administered to male cynomolgus monkeys at the age of 4 to 5 to make Parkinson's disease model cynomolgus monkeys. MPTP was additionally administered to animals with neurologic deficit scores (Neurological deficit scores: NDSs, Schneider et al., "Development of levodopa-induced dyskinesias in parkinsonian monkeys may depend upon rate of symptom onset and/or duration of symptoms", Brain Res., 2003, 990: 38-44.) of less than 25.

Eight Parkinson's disease model cynomolgus monkeys were randomized into 5 groups of approximately equal mean and variance in terms of Parkinson behavior rating (Tremor score) and body weight. On subsequent cycle 2, cycle 3, cycle 4 and cycle 5, the animals were crossed over to receive the other treatments. A wash out period of one week or more was set between each cycle.

By the end of the study, each animal had received all the five treatments. Istradefylline is an adenosine A_{2A} receptor antagonist approved as a drug for improving a wearing off phenomenon in Parkinson's disease being treated with a levodopa-containing preparation in the United States of America and Japan (Patent Literature 4). As a vehicle, an aqueous solution containing 0.43% (w/v) methyl cellulose 400, 10% (v/v) dimethyl sulfoxide (DMSO), and 4.5% (w/v) Cremophor(R) was used.

**[Table 1]**

| Group | Administered substance | Dose (mg/kg) |
|---|---|---|
| G1 | Vehicle | - |
| G2 | Compound (I) low dose | 5 |
| G3 | Compound (I) middle dose | 15 |
| G4 | Compound (I) high dose | 50 |
| G5 | Istradefylline | 60 |

Before the administration, and 1 hr., 4 hrs., and 24 hrs. after the administration of the test substances, the NDSs of the monkeys were evaluated. The evaluation items are as follows. The behavior evaluation was performed in a blinded manner. The NDSs of the groups were compared using Steel's test. The p-values of less than 0.05 were considered to be statistically significant.

### Subgroup 1: generalized behaviors (0 - 32)

1.1a Appetite
   0: Normal
   1: Up to 30% food left
   2: Up to 65% food left
   3: Greater than 65% food left
1.1b Response to offered food
   0: Responds to offered food
   1: Inconsistent response to offered food
   2: Little to no interest in offered food
1.2 Overall activity
   0: Normal
   1: Mild hypokinesia; decrease in spontaneous movement
   2: Moderate hypokinesia; sparse spontaneous movement
   3: Severe hypokinesia; no spontaneous movement; tends to remain in one place
1.3 Appearance
   0: Normal
   1: Mild but noticeable decrease in self-maintenance
   2: Moderate decrease in self-maintenance
   3: Severe lack of self-maintenance
1.4 Posture
   0: Normal
   1: Minor flexing or stooping
   2: Moderate flexing or stooping; head and upper body almost parallel to floor of cage
   3: Severe flexing or stooping; head lowered to feet
1.5 Balance
   0: Normal
   1: Mild loss on arising or movement
   2: Moderate loss on arising or movement; occasionally falls or supports self to keep from falling
   3: Severe loss of balance; falls
1.6 Climbing
   0: Climbing observed with no delays in initiation/execution
   1: Mild deficit in ability to climb and support weight
   2: Moderate deficit in ability to climb and support weight; tends to 'slide down'
   3: Absence of any climbing behavior
1.7 Tremor
   0: Absent
   1: Slight and intermittent
   2: Slight and persistent or moderate and intermittent
   3: Moderate/severe and persistent
1.8 Freezing during movement
   0: Absent
   1: Occasional freezing for brief periods
   2: Occasional freezing for prolonged periods or frequent freezing for brief periods
   3: Prolonged and frequent freezing
1.9 Facial expression
   0: Normal
   1: Mild hypomimia
   2: Moderate hypomimia
   3: Severe, masked fixed faces
1.10 Defense reaction
   0: Normal
   1: Mild, noticeable reduction in reaction or slowed reaction, but can be provoked to react
   2: Moderate reduction in reaction; can produce facial display but not body reaction
   3: Little to no response

### Subgroup 2: specific motor functions (0 - 21)

2.1a Upper limb movement
   0: Normal
   1: Noticeable decrease of upper limb use
   2: Moderate decrease of upper limb use
   3: Severe decrease of upper limb use
2.1b Lower limb movement
   0: Normal
   1: Noticeable decrease of lower limb use
   2: Moderate decrease of lower limb use
   3: Severe decrease of lower limb use
2.2a Range of upper limb movement
   0: Normal
   1: Mild reduction, but able to extend limb almost completely
   2: Moderate reduction in range of motion with some usage difficulty; exhibits definite flexor posturing of limbs at rest
   3: Severe reduction; flexor posturing of limbs at rest
2.2b Range of lower limb movement
   0: Normal
   1: Mild reduction, but able to extend limb almost completely
   2: Moderate reduction in range of motion with some usage difficulty; exhibits definite flexor posturing of limbs at rest
   3: Severe reduction; flexor posturing of limbs at rest
2.3 Fine motor skills
   0: Normal
   1: Noticeable clumsiness in ability to grasp food or rewards; occasional dropping of food; can grasp food
   2: Moderate decrease in ability to grasp and handle food; drops food often; uses two hands to bring food to mouth
   3: Unable to consistently grasp, hold or manipulate food; may bring mouth directly to food
2.4 Eating
   0: Normal
   1: Mild slow chewing and/or occasional pauses
   2: Mild slow chewing with frequent pauses or moderately slow chewing with occasional pauses
   3: No effective eating on own
2.5 Eye blink rate (number of blinks in 20 sec)
   0: Normal (6 or more blinks)
   1: Mildly reduced (4-5 blinks)
   2: Moderately reduced (2-3 blinks)
   3: Severely reduced (0 - 1 blinks)

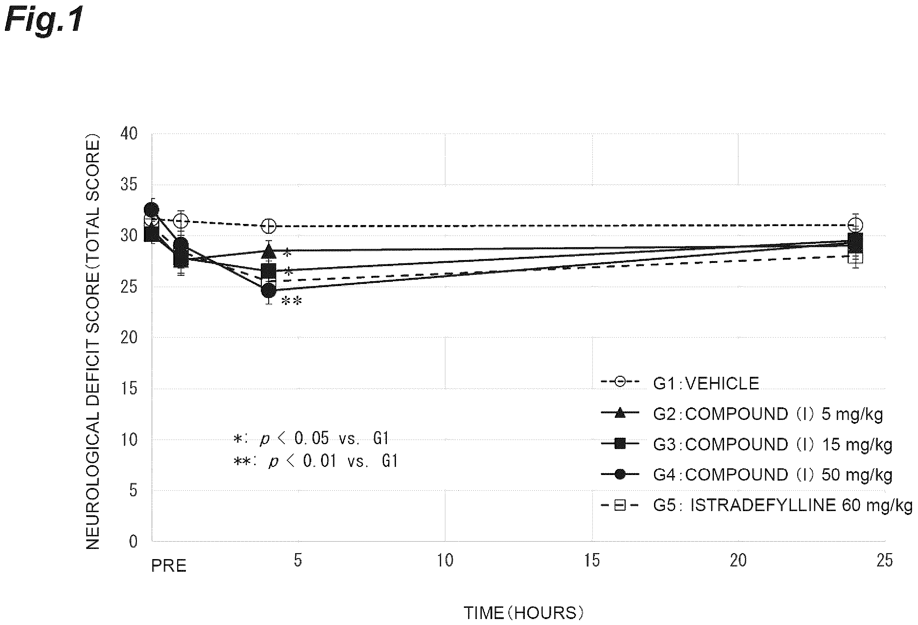

The total scores of the NDSs (total scores of subgroup 1 and subgroup 2) were shown in Table 2 and Figure 1. In middle-dose and high-dose Compound (I)-treated groups (G3 and G4) and an istradefylline-treated group (G5), the total scores 4 hrs. after each dosing were significantly lower as compared with the vehicle-treated group (G1 vs. G3: p = 0.0215, G1 vs. G4: p = 0.0135, G1 vs. G5: p = 0.0048).

**[Table 2]**

| Total score | | | | |
|---|---|---|---|---|
| Group | Pre | 1 hr. | 4 hrs. | 24 hrs. |
| G1 | 31.6±0.9 | 31.4±1.0 | 30.9±0.7 | 31.0±1.1 |
| G2 | 30.6±1.1 | 27.6±1.5 | 28.5±1.0 | 29.0±1.0 |
| G3 | 30.1±0.9 | 27.8±1.5 | 26.5±1.0* | 29.5±1.1 |
| G4 | 32.5±1.1 | 29.1±1.3 | 24.6±1.3* | 29.3±1.6 |
| G5 | 30.9±1.3 | 28.4±1.6 | 25.4±1.0** | 28.0±1.2 |

| | | | | |
|---|---|---|---|---|
| ***: *p* < 0.05 vs. G1, **: *p* < 0.01 vs. G1 | | | | |

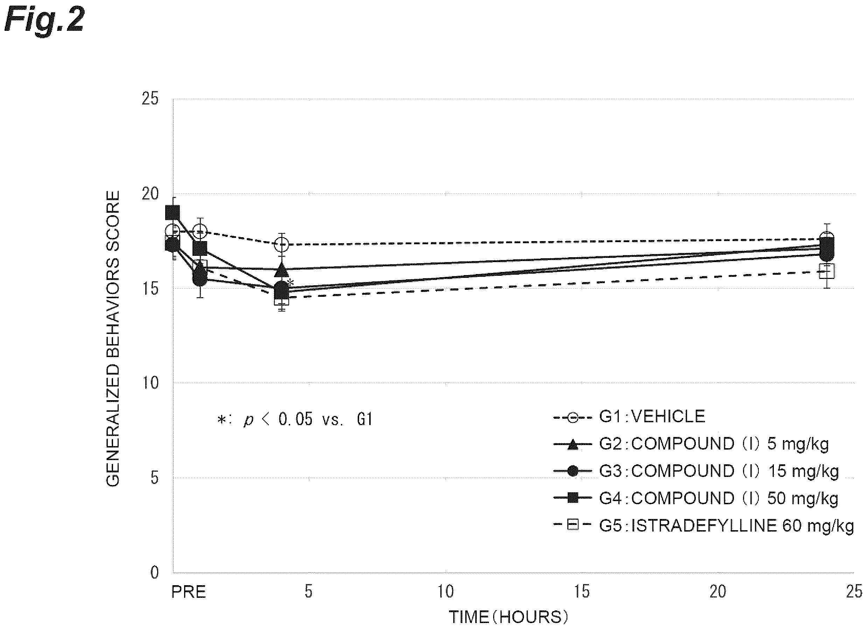

The generalized behaviors scores were shown in Table 3 and Figure 2. In the istradefylline-treated group (G5), the generalized behaviors score 4 hrs. after dosing was significantly lower as compared with the vehicle-treated group (G1 vs. G5: p = 0.0327).

**[Table 3]**

| Subgroup 1: GENERALIZED BEHAVIORS | | | | |
|---|---|---|---|---|
| Group | Pre | 1 hr. | 4 hrs. | 24 hrs. |
| G1 | 18.0±0.7 | 18.0±0.7 | 17.3±0.6 | 17.6±0.8 |
| G2 | 17.4±0.8 | 16.1±0.9 | 16.0±0.7 | 17.1±0.8 |
| G3 | 17.3±0.6 | 15.5±1.0 | 15.0±0.8 | 16.8±0.9 |
| G4 | 19.0±0.8 | 17.1±0.8 | 14.8±1.0 | 17.3±1.1 |
| G5 | 17.4±0.9 | 16.1±0.9 | 14.5±0.6* | 15.9±0.9 |

| | | | | |
|---|---|---|---|---|
| *: *p* < 0.05 vs. G1 | | | | |

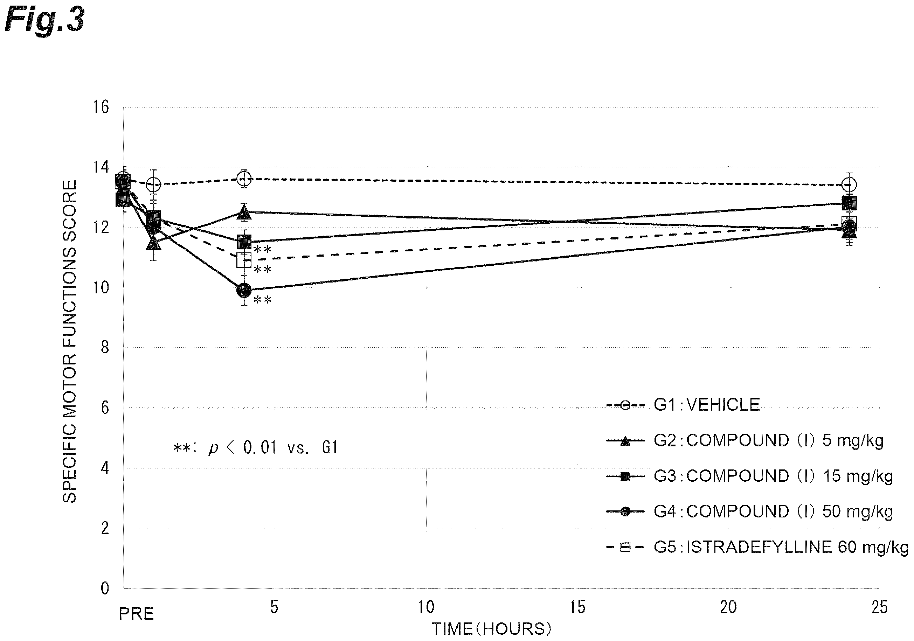

The scores of the specific motor functions were shown in Table 4 and Figure 3. In the middle-dose and high-dose Compound (I)-treated groups (G3 and G4) and the istradefylline-treated group (G5), the scores of the specific motor functions 4 hrs. after each dosing were significantly lower as compared with the vehicle-treated group (G1 vs. G3: p = 0.0089, G1 vs. G4: p = 0.0025, G1 vs. G5: p = 0.0037).

**[Table 4]**

| Subgroup 2: Specific motor functions | | | | |
|---|---|---|---|---|
| Group | Pre | 1 hr. | 4 hrs. | 24 hrs. |
| G1 | 13.6±0.4 | 13.4±0.5 | 13.6±0.3 | 13.4±0.4 |
| G2 | 13.3±0.5 | 11.5±0.6 | 12.5±0.3 | 11.9±0.4 |
| G3 | 12.9±0.4 | 12.3±0.5 | 11.5±0.4** | 12.8±0.3 |
| G4 | 13.5±0.4 | 12.0±0.5 | 9.9±0.5** | 12.0±0.6 |
| G5 | 13.5±0.4 | 12.3±0.8 | 10.9±0.5** | 12.1±0.5 |

| | | | | |
|---|---|---|---|---|
| **: *p* < 0.01 vs. G1 | | | | |

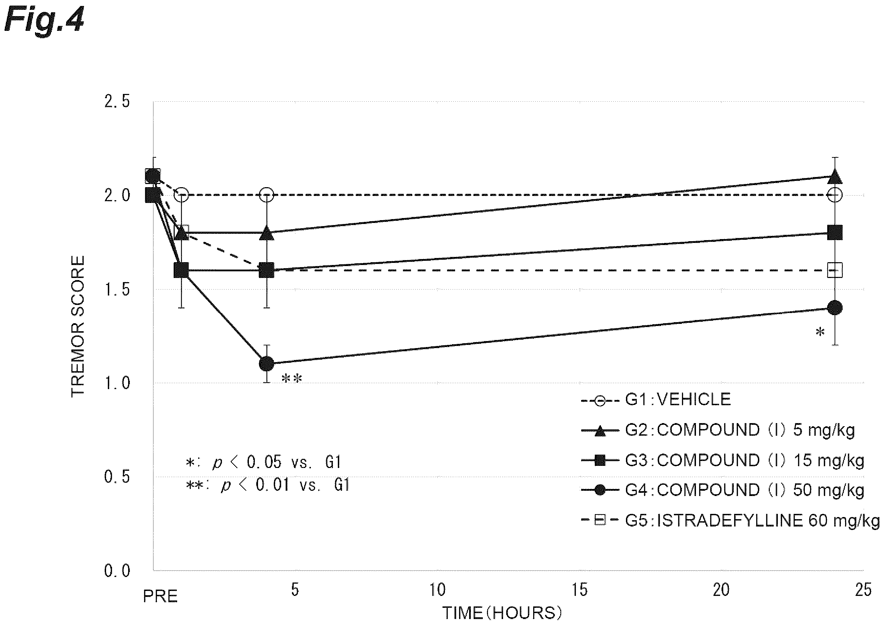

The tremor scores were shown in Table 5 and Figure 4. In the high-dose Compound (I)-treated group (G4), the tremor scores 4 hrs. and 24 hrs. after dosing were significantly lower as compared with the vehicle-treated group (G1 vs. G4 after 4 hrs.: p = 0.0024, G1 vs. G4 after 24 hrs.: p = 0.0317).

**[Table 5]**

| Tremor score | | | | |
|---|---|---|---|---|
| Group | Pre | 1 hr. | 4 hrs. | 24 hrs. |
| G1 | 2.1±0.1 | 2.0±0.0 | 2.0±0.0 | 2.0±0.0 |
| G2 | 2.0±0.0 | 1.8±0.2 | 1.8±0.2 | 2.1±0.1 |
| G3 | 2.0±0.0 | 1.6±0.2 | 1.6±0.2 | 1.8±0.2 |
| G4 | 2.1±0.1 | 1.6±0.2 | 1.1±0.1** | 1.4±0.2* |
| G5 | 2.1±0.1 | 1.8±0.2 | 1.6±0.2 | 1.6±0.2 |

| | | | | |
|---|---|---|---|---|
| ***: *p* < 0.05 vs. G1, **: *p* < 0.01 vs. G1 | | | | |

### Test Example 2: Therapeutic effect of combined administration of Compound (I) and levodopa

Parkinson's disease model cynomolgus monkeys were provided in the same way as in Test Example 1, and the therapeutic effect of the combined administration of Compound (I) and levodopa was examined.

By the end of the study, each animal had received all the five treatments. As a vehicle, an aqueous solution containing 0.43% (w/v) methyl cellulose 400, 10% (v/v) dimethyl sulfoxide (DMSO), and 4.5% (w/v) Cremophor(R) was used.

**[Table 6]**

| Group | Administered substance and dose |
|---|---|
| G1 | Vehicle |
| G2 | Levodopa 10 mg/kg |
| G3 | Levodopa 5 mg/kg |
| G4 | Levodopa 5 mg/kg + Compound (I) 50 mg/kg |
| G5 | Levodopa 5 mg/kg + Compound (I) 25 mg/kg |

Before the administration, and 1 hr., 4 hrs., and 24 hrs. after the administration of the test substances, the NDSs of the monkeys were evaluated. The evaluation items and the evaluation methods are the same as in Test Example 1.

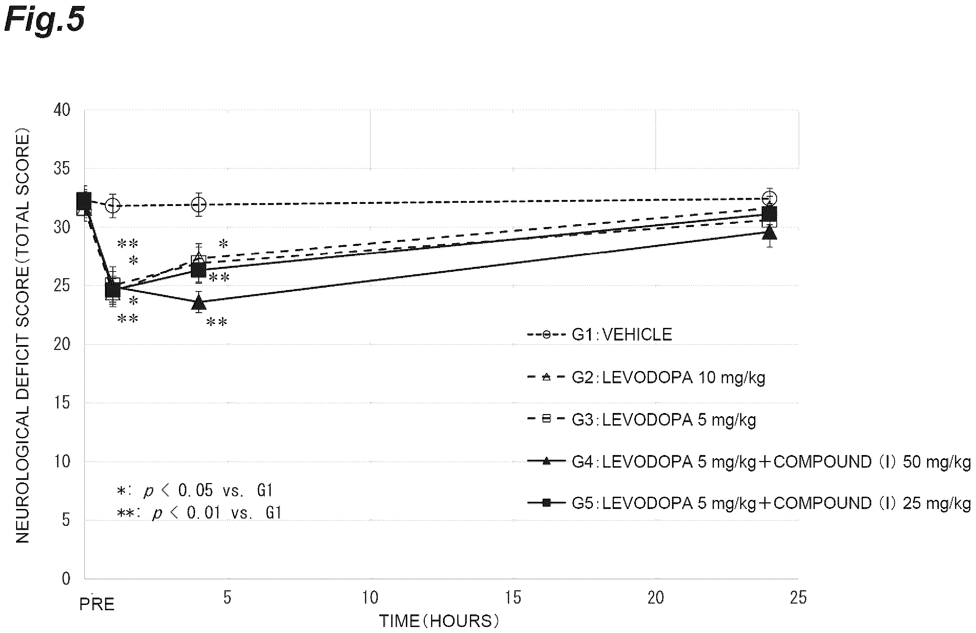

The total scores of the NDSs were shown in Table 7 and Figure 5. In the levodopa-treated groups (G2 to G3) and levodopa + Compound (I)-treated groups (G4 to G5), the total scores 1 hr. after each dosing were significantly lower as compared with the vehicle-treated group (G1 vs. G2: p = 0.0049, G1 vs. G3: p = 0.0293, G1 vs. G4: p = 0.0186, G1 vs. G5: p = 0.0048). In the 10 mg/kg of levodopa-treated group (G2) and the levodopa + Compound (I)-treated groups (G4 to G5), the total scores 4 hrs. after each dosing were significantly lower as compared with the vehicle-treated group (G1 vs. G2: p = 0.0154, G1 vs. G4: p = 0.0033, G1 vs. G5: p = 0.0081).

**[Table 7]**

| Total score | | | | |
|---|---|---|---|---|
| Group | Pre | 1 hr. | 4 hrs. | 24 hrs. |
| G1 | 32.3±0.9 | 31.8±1.0 | 31.9±1.0 | 32.4±0.9 |
| G2 | 31.6±0.8 | 24.4±1.2** | 27.3±1.0* | 31.6±1.0 |
| G3 | 31.6±1.1 | 25.0±1.6* | 26.9±1.7 | 30.6±1.1 |
| G4 | 32.1±0.9 | 24.9±1.3* | 23.6±0.9** | 29.6±1.3 |
| G5 | 32.3±1.2 | 24.6±1.2** | 26.3±1.0** | 31.1±0.9 |

| | | | | |
|---|---|---|---|---|
| *: *p* < 0.05 vs. G1, **: *p* < 0.01 vs. G1 | | | | |

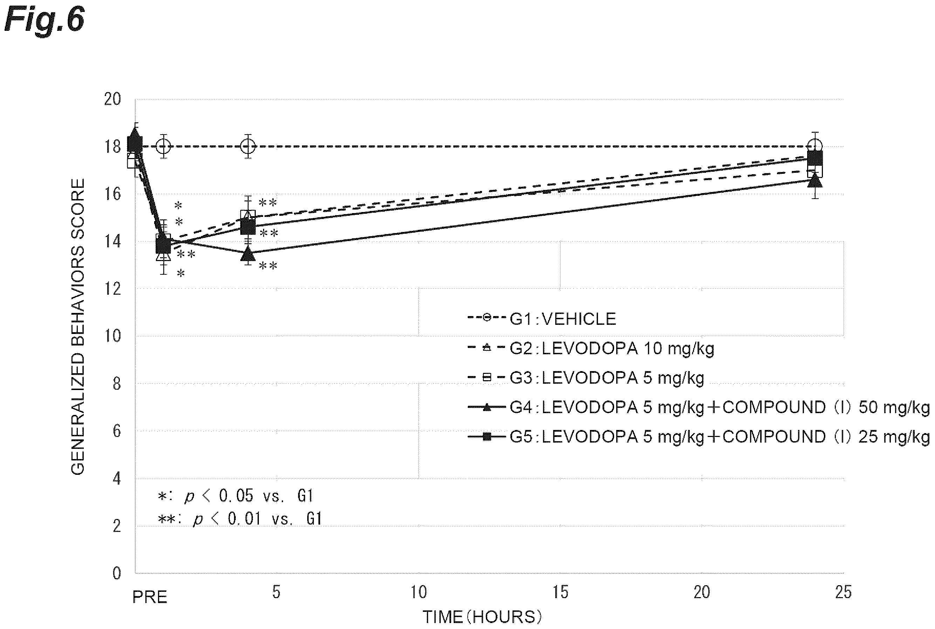

The generalized behaviors scores were shown in Table 8 and Figure 6. In the levodopa-treated groups (G2 to G3) and the levodopa + Compound (I)-treated group (G4 to G5), the generalized behaviors scores 1 hr. after each dosing were significantly lower as compared with the vehicle-treated group (G1 vs. G2: p = 0.0128, G1 vs. G3: p = 0.0127, G1 vs. G4: p = 0.0309, G1 vs. G5 p = 0.0051). In the 10 mg/kg of levodopa-treated group (G2) and the levodopa + Compound (I)-treated groups (G4 to G5), the generalized behaviors scores 4 hrs. after each dosing were significantly lower as compared with the vehicle-treated group (G1 vs. G2: p = 0.0098, G1 vs. G4 p = 0.0027, G1 vs. G5: p = 0.0084).

**[Table 8]**

| Subgroup 1: GENERALIZED BEHAVIORS | | | | |
|---|---|---|---|---|
| Group | Pre | 1 hr. | 4 hrs. | 24 hrs. |
| G1 | 18.0±0.5 | 18.0±0.5 | 18.0±0.5 | 18.0±0.6 |
| G2 | 17.8±0.6 | 13.5±0.9* | 15.0±0.7** | 17.6±0.7 |
| G3 | 17.4±0.7 | 14.0±0.7* | 15.0±0.9 | 17.0±0.7 |
| G4 | 18.5±0.5 | 14.1±0.8* | 13.5±0.5** | 16.6±0.8 |
| G5 | 18.1±0.7 | 13.8±0.8** | 14.6±0.7** | 17.5±0.8 |

| | | | | |
|---|---|---|---|---|
| *: *p* < 0.05 vs. G1, **: *p* < 0.01 vs. G1 | | | | |

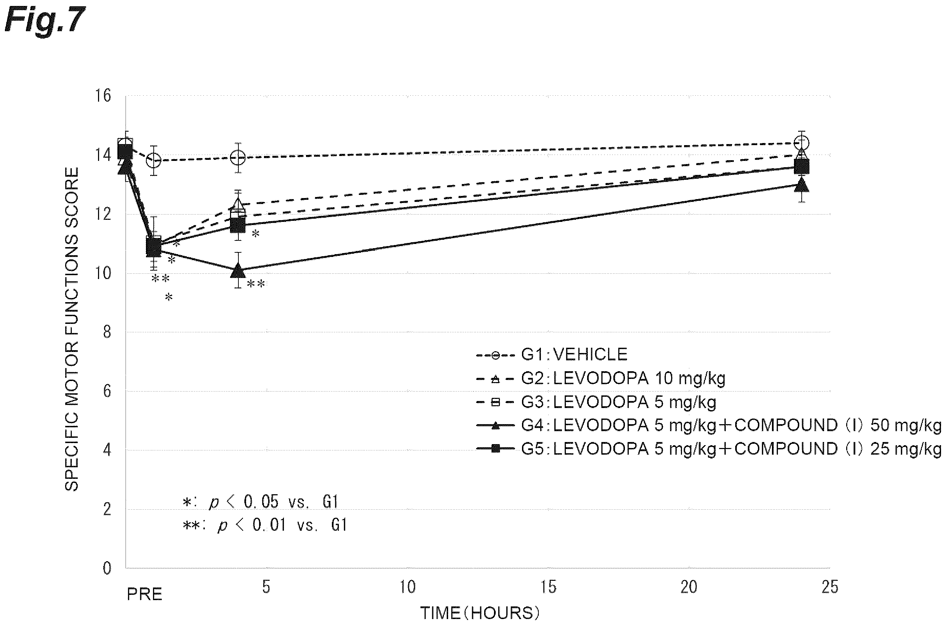

The scores of the specific motor functions were shown in Table 9 and Figure 7. In the levodopa-treated groups (G2 to G3) and the levodopa + Compound (I)-treated groups (G4 to G5), the specific motor functions scores 1 hr. after each dosing were significantly lower as compared with the vehicle-treated group (G1 vs. G2: p = 0.0099, G1 vs. G3: p = 0.0366, G1 vs. G4 p = 0.0177, G1 vs. G5 p = 0.0175). In the levodopa + Compound (I)-treated group (G4 to G5), the specific motor functions scores 4 hrs. after each dosing were significantly lower as compared with the vehicle-treated group (G1 vs. G4: p = 0.0080, G1 vs. G5: p = 0.0366).

**[Table 9]**

| Subgroup 2: | Specific motor functions | | | |
|---|---|---|---|---|
| Group | Pre | 1 hr. | 4 hrs. | 24 hrs. |
| G1 | 14.3±0.5 | 13.8±0.5 | 13.9±0.5 | 14.4±0.4 |
| G2 | 13.9±0.4 | 10.9±0.5** | 12.3±0.5 | 14.0±0.5 |
| G3 | 14.3±0.5 | 11.0±0.9* | 11.9±0.8 | 13.6±0.5 |
| G4 | 13.6±0.5 | 10.8±0.6* | 10.1±0.6** | 13.0±0.6 |
| G5 | 14.1±0.5 | 10.9±0.5* | 11.6±0.5* | 13.6±0.3 |

| | | | | |
|---|---|---|---|---|
| *: *p* < 0.05 vs. G1, **: *p* < 0.01 vs. G1 | | | | |

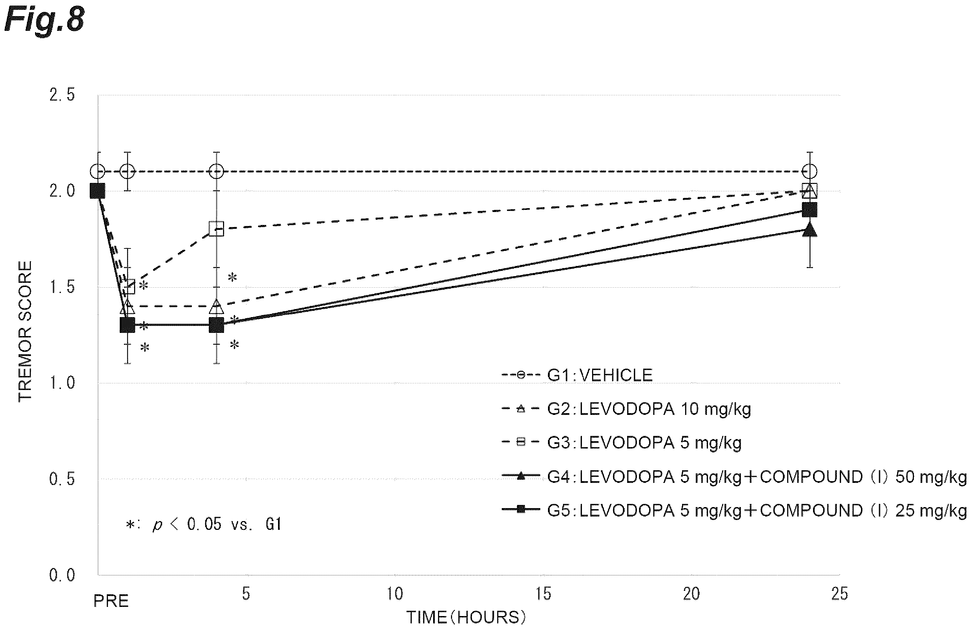

The tremor scores were shown in Table 10 and Figure 8. In the 10 mg/kg of levodopa-treated group (G2) and the levodopa + Compound (I)-treated groups (G4 to G5), the tremor scores 1 hr. after each dosing were significantly lower as compared with the vehicle-treated group (G1 vs. G2: p = 0.0287, G1 vs. G4: p = 0.0103, G1 vs. G5: p = 0.0103). In the 10 mg/kg of levodopa-treated group (G2) and the levodopa + Compound (I)-treated groups (G4 to G5), the tremor scores 4 hrs. after each dosing were significantly lower as compared with the vehicle-treated group (G1 vs. G2: p = 0.0287, G1 vs. G4: p = 0.0103, G1 vs. G5: p = 0.0103).

**[Table 10]**

| Tremor score | | | | |
|---|---|---|---|---|
| Group | Pre | 1 hr. | 4 hrs. | 24 hrs. |
| G1 | 2.1±0.1 | 2.1±0.1 | 2.1±0.1 | 2.1±0.1 |
| G2 | 2.0±0.0 | 1.4±0.2* | 1.4±0.2* | 2.0±0.0 |
| G3 | 2.0±0.0 | 1.5±0.2 | 1.8±0.2 | 2.0±0.0 |
| G4 | 2.0±0.0 | 1.3±0.2* | 1.3±0.2* | 1.8±0.2 |
| G5 | 2.0±0.0 | 1.3±0.2* | 1.3±0.2* | 1.9±0.1 |

| | | | | |
|---|---|---|---|---|
| *: *p* < 0.05 vs. G1 | | | | |

### Test Example 3: Effect of Compound (I) on levodopa-induced dyskinesia

A levodopa/carbidopa mixture containing 80 mg/kg of levodopa and 20 mg/kg of carbidopa and having a solution volume of 5 mL/kg was orally administered to Parkinson's disease model cynomolgus monkeys at the age of 6 to 7 prepared in the same way as in Test Example 1 for 8 consecutive days to provide levodopa-induced dyskinesia model cynomolgus monkeys. The induction of dyskinesia was determined based on the "dyskinesia score" in accordance with the following behavior evaluation items in the following test on activity in home cages, and animals having dyskinesia scores of 1 or more on day 7 of the repeated administration of the levodopa/carbidopa mixture were used.

### Home cage activities corresponding to dyskinesia scores

0: Normal home cage activity (exploring or inactivity, able to drink water, plays with mirror or caging, climbing of the walls).
1: Some interference with normal activity, however able to climb walls.
2: Occasional exploration of cage, some stereotypy
3: Little normal movement, lack of climbing of walls, clear stereotypy
4: Lack of normal home cage activity, lack of normal exploratory behavior and movement, severe stereotypy

Seven levodopa-induced dyskinesia model cynomolgus monkeys were randomized into 4 groups of approximately equal mean and variance in terms of body weight. On subsequent cycles 2, 3, and 4, the animals were crossed over to receive the other treatment. A wash out period of one week or more was set between cycles.

One of the seven animals was excluded from the evaluation of cycles 3 and 4 because it was unable to get up due to dyskinesia on day 8 in cycle 3 of the repeated administration of the levodopa/carbidopa mixture.

By the end of the study, each animal had received all the four treatments. Each substance was administered 1 hr. after the administration on day 8 of the repeated administration of the levodopa/carbidopa mixture. As a vehicle, an aqueous solution containing 0.43% (w/v) methyl cellulose 400,10% (v/v) dimethyl sulfoxide (DMSO), and 4.5% (w/v) Cremophor(R) were used.

**[Table 11]**

| Group | Administered substance and dose |
|---|---|
| G1 | Vehicle |
| G2 | Compound (I) 25 mg/kg |
| G3 | Compound (I) 50 mg/kg |
| G4 | Istradefylline 60 mg/kg |

Before the administration, and 1 hr., 4 hrs., and 24 hrs. after the administration of the test substances, the dyskinesia scores of the animals were evaluated. The behavior evaluation was performed in a blinded manner. The dyskinesia scores of the groups were compared using Steel's test. The p-values of less than 0.05 were considered to be statistically significant.

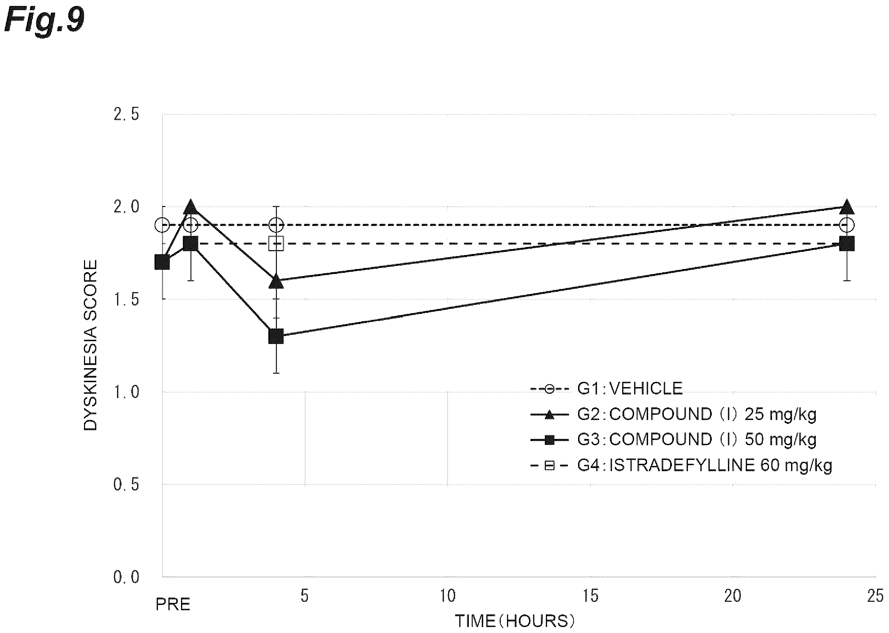

The dyskinesia scores were shown in Table 12 and Figure 9. The statistically significant differences between the four groups were not observed at any measurement time. The mean value of the dyskinesia scores 4 hrs. after the administration in Compound (I)-treated group (G3) was however lower as compared with the vehicle-treated group (G1) and the istradefylline-treated group (G4) at the same measurement time.

**Table 12]**

| Group | Pre | 1 hr. | 4 hrs. | 24 hrs. |
|---|---|---|---|---|
| G1 | 1.9±0.1 | 1.9±0.1 | 1.9±0.1 | 1.9±0.1 |
| G2 | 1.7±0.2 | 2.0±0.0 | 1.6±0.2 | 2.0±0.0 |
| G3 | 1.7±0.2 | 1.8±0.2 | 1.3±0.2 | 1.8±0.2 |
| G4 | 1.7±0.2 | 1.8±0.2 | 1.8±0.2 | 1.8±0.2 |

## Claims

1. A pharmaceutical composition for treating Parkinson's disease, comprising N-[(1S)-2,2,5,7-tetrafluoro-2,3-dihydro-1H-inden-1-yl]sulfamide represented by formula (I): 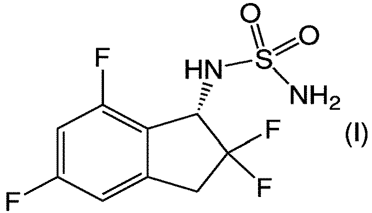 or a pharmaceutically acceptable salt thereof.

2. The pharmaceutical composition according to claim 1, wherein the composition is administered in combination with a dopamine-replacement-therapeutic agent.

3. The pharmaceutical composition according to claim 2, wherein the dopamine-replacement-therapeutic agent comprises levodopa.

4. The pharmaceutical composition according to any one of claims 1 to 3, for treating a motor symptom of Parkinson's disease.
